# EUROPEAN PATENT SPECIFICATION

(11) **EP 0 590 060 B1**
(45) Date of publication and mention of the grant of the patent: **17.09.1997**
(21) Application number: 92914240.4
(22) Date of filing: 11.06.1992
(51) Int. Cl.: A61K 9/52

(54) **ORALLY ADMINISTRABLE THERAPEUTIC PROTEINS AND METHOD OF MAKING**
ORAL VERABREICHBARE THERAPEUTISCHE PROTEINE UND HERSTELLUNGSVERFAHREN
PROTEINES THERAPEUTIQUES ADMINISTRABLES PAR VOIE ORALE ET LEUR PROCEDE DE PRODUCTION

(30) Priority: 21.06.1991 US 719160
(43) Date of publication of application: 06.04.1994
(73) Proprietor: UNIVERSITY OF CINCINNATI, Cincinnati, Ohio 45221-0627 (US)
(72) Inventor: MICHAEL, Jacob G., Cincinnati, OH 45215 (US)
(74) Representative: Findlay, Alice Rosemary
(86) International application number: PCT/US92/05004
(87) International publication number: WO 93/00077

(56) References cited:
- EP-A- 0 035 780
- EP-A- 0 192 321
- EP-A- 0 277 741
- EP-A- 0 278 877
- US-A- 4 348 384
- US-A- 4 642 232
- US-A- 4 774 226
- US-A- 4 820 627
- US-A- 5 049 390

## Description

### Background of the Invention

Numerous polypeptides are used in treatment and prevention of diseases. These can include proteins, protein fragments and related biomolecules.

Numerous polypeptide molecules cause an allergic reaction in man and animals. Hay fever, more particularly ragweed sensitivity is characterized by itching of the mucosa of the nose, mouth, pharynx and eyes, lacrimation, sneezing, watery nasal discharge, coughing and sometimes asthmatic wheezing and afflicts a considerable number of humans. Sensitivity to bee venom and drugs is characterized classically by a wheal and flare reaction, swelling, anaphylaxis and occasionally death. Food allergies are manifested by urticaria, nausea, diarrhea, and hives.

The appearance of allergy or atopy is involved with production of a tissue-sensitizing immunoglobulin, IgE antibody. These antibodies have an affinity for receptors on cells in various body tissues. The receptors on mast cells and basophils are of special significance since these cells contain pharmacologically-active mediators, such as histamine, serotonin, and kinins concentrated in cytoplasmic granules. When IgE antibodies are fixed to mast cells and basophils, contact with the sensitizing antigens can result in cross-linking. This cross-linking causes degranulation of mast cells and basophils which in turn release the pharmacologically active mediators (particularly histamine) and which are responsible for manifestations of the allergic response.

Thus, these disturbances are caused by immunoglobulin E reacting with specific polypeptide molecules, the allergens. Untreated, these disturbances can lead to asthma in the case of hayfever, disabling reactions in the case of insect venom and limited dietary intake in the case of foods. Treatment for hayfever usually entails trial of orally active antihistamines, sometimes in combination with sympathomimettes such as phenylpropanolamine or phenylephrine. The symptoms may thus be partially suppressed, but the basic physiological difficulty, the histamine release due to IgE antibody crosslinking by the allergens persists.

Pharmacological treatment of insect stings is difficult since it is not possible to know when the event may occur and the reaction can be very severe if treatment is not immediate. Similarly the food reaction must have immediate treatment to achieve its effect. Therefore, there is a need for a more fundamental treatment, namely desensitization.

One of the conventional and widely used methods of treating allergy is by immunizing or desensitizing a patient by repeated injections of small, gradually increasing amounts of the allergen, which is believed to increase the levels of blocking immunoglobulin G (IgG) antibodies and at the same time arresting the production of IgE antibodies (immunotherapy). This apparently conflicting effect of allergen administration is not fully understood but apparently involves differences in immunoregulation of two different classes of antibodies, viz, IgG and IgE.

There are typically many ways of administering allergy treatments. Allergens can be administered intranasally, by injection, or orally. From the patient's point of view, the preferred method of administration, is orally.

Oral administration of the allergen thus far was found to be quite ineffective. The allergen can have a negative effect on the bucal, oesophagael and tracheal tissue. More likely, however, the protein which makes up the allergen is destroyed by gastric and intestinal juices. In order for it to be effective, it must reach the lymphoid tissue in the small intestine. If destroyed prior to this, it has no effect.

Digestion by gastrointestinal enzymes is also encountered with other peptides, and proteins including vaccines and hormones. Again, this prevents preparation of effective orally administrable vaccines and hormones.

There have been many attempts to overcome this problem. The primary method is the enteric coating wherein the allergen can be microencapsulated with a coating which dissolves in basic pHs. Thus, the coated particle can pass through the stomach and is released upon reaching the small intestines where the pH changes from acid to base. Even under these circumstances, the allergen does not remain effective since the digestive juices within the small intestines can act to destroy the protein.

Further, the microencapsulation process itself can destroy the protein. The microencapsulation process requires significant mixing, heating and spraying of the various components. The physical forces generated during this process can denature the protein molecule.

Finally, microencapsulation techniques have employed nonaqueous solvents such as ethanol and other organic solvents. These also decrease the efficacy of the microencapsulation of the protein.

These problems occur with oral administration of most therapeutic proteins.

US Patent 4348384 describes a pharmaceutical comprising coagulation factor VIII or IX and a protease inhibitor which are encapsulated in intestine capsules. The joints of the intestine capsules are sealed by treating them with a solution of suitable material in acetone.

US Patent 4642232 describes a formulated preparation containing an immunotherapeutically active amount of at least one allergen together with an anti-allergic drug. The preparation is placed in a capsule and coated with a non-aqueous enteric coating.

EP-A-0192321 describes an enteric-coated allergen which in the described examples are formed by use of an organic solvent coating system.

EP-A-0277741 describes the formation of spherical granules comprising a core of an analgesic.

US Patent 4820627 describes a method of producing a diagnostic reagent in tablet form.

### Summary of the Invention

A method of forming a microencapsulated allergy treatment pharmaceutical comprises, in accordance with the invention, forming an aqueous solution of a therapeutic protein, the therapeutic protein comprising an allergen involved in production of a tissue sensitizing immunoglobulin IgE antibody, coating the aqueous solution of protein onto inert particles and drying the particles, and applying an aqueous emulsion of an enteric coating onto the coated particles and subsequently drying the coated particles.

The present invention is premised on the realization that an orally administrable protein can be formed by combining the protein with a stabilizing agent which is a therapeutically inactive protein. The present invention is also premised on the realization that an orally administrable therapeutic protein can be formed by combining a therapeutic protein such as an allergen along with a stabilizing agent and microencapsulating the combination with a coating which is insoluble under acid conditions as would be encountered by the drug passing through the stomach.

The present invention is further premised on the realization that by microencapsulating the protein under totally aqueous conditions without employing any nonaqueous solvents, the structure and the immunogenicity of the protein remain intact.

More particularly, the present invention is premised on the realization that an allergen, such as ragweed or the like combined with an stabilizing agent, such as ovalbumin, when coated onto a nonpareil and subsequently microencapsulated with an enteric coating, form microcapsules which are suitable to provide an orally administrable antigenic composition. These can pass through the stomach, be released in the small intestines and express their immunogenic activity.

The stabilizing agent is a therapeutically inactive water soluble protein. Bovine serum albumin and gelatin are both suitable and will act to prevent denaturing or fragmentation of the therapeutic protein.

The stabilizing agent preferably is a proteolytic enzyme inhibiting protein which assists in the maintenance of the activity of the protein during microencapsulation and in the subsequent release in the small intestines. The stabilizing agent protects the protein in the small intestines permitting it to reach the lymphoid tissue in the small intestines and thus act upon the immune system.

The objects and advantages of the present invention will be further appreciated in light of the following detailed description and drawings.

### Brief Description of Drawings

Fig. 1 is a graph showing antibody titres of mice fed OVA; and
Fig. 2 is a graph showing antibody titres over time of mice treated with various compositions.

### Detailed Description

According to the present invention, an orally administrable therapeutic protein is formed by combining a therapeutically active protein with a stabilizing agent in the proportions discussed below. These can be combined in dry form with fillers and administered in a gel capsule. It is preferable to microencapsulate the combination with an enteric coating.

The therapeutic proteins are combined with the stabilizing agent which is an inactive protein in an aqueous solution. The aqueous solution is then microencapsulated in a water dispersed polymer by spraying the protein solution onto nonpareils and coating the particles with a water emulsion polymer which upon solidification is acid resistant. This protects the therapeutic protein as it passes through the stomach and releases it in the small intestines where it can act upon the Peyers patches, i.e., the lymphoid tissue in the small intestines.

The stabilizing agent acts to protect the therapeutic protein during the coating process promotes adhesion onto nonparails and in a preferred embodiment acts as a proteolytic enzyme inhibitor in the small intestine.

For purposes of the present invention, therapeutic proteins will include allergenic proteins and digested fragments thereof. These include pollen allergens from ragweed, rye, June grass, orchard grass, sweet vernal grass, red top, timothy, yellow dock, wheat, corn, sagebrush, blue grass, California annual grass, pigweed, Bermuda grass, Russian thistle, mountain cedar, oak, box elder, sycamore, maple, elm, etc., dust and mites, bee venom, food allergens, animal dander, and other insect venoms.

Further, any of these allergens digested, according to the method disclosed in U.S. Patent 4,469,677, are also suitable for use in the present invention. This basically discloses the proteolytic enzymatic digestion of allergens. Accordingly, polypeptides formed by such proteolytic enzymatic digestion are also suitable for use as therapeutic proteins for use in the present invention.

Other therapeutic proteins include microbial vaccines such as viral, bacterial and protozoal vaccines. These include vaccines which are glycoproteins. Other therapeutic proteins suitable for use in the present invention would also include human growth factor, myelin basic proteins, collagen S antigen, transforming growth factor beta.

The stabilizing agent can provide physical protection for the therapeutic protein and preferably will provide physiological protection by inhibiting the formation or the activity of proteolytic enzymes typically encountered in the human intestine.

Stabilizing agents which provide physical protection are therapeutically inactive water soluble proteins such as bovine serum albumin and gelatin. These act to protect the therapeutic protein during the coating process and during administration.

Preferable stabilizing agents for use in the present invention are inhibitors of proteolytic enzymes typically found in gastric juices. Trypsin inhibitors are particularly useful. Specific stabilizing agents include ovalbumin, aprotinin, bestatin, leupeptin, alpha₂ macroglobulin, pepstatin, as well as soybean proteins. For use in the present invention, ovalbumin and soybean protein are preferred.

To form orally administrable microcapsules for use in the present invention, an aqueous solution of the therapeutic protein, stabilizing agent and optionally a binding agent is first formed. Generally there will be at least equal molar amounts of the stabilizing agent and therapeutic protein. However, it is preferred to have a stoichiometric excess of the stabilizing agent. Preferably a 10:1 molar ratio of the stabilizing agent to therapeutic protein can be employed. Even a greater excess should be employed if desired. But when the concentration of protein becomes too excessive, the solution may become excessively viscous.

The aqueous solution will include generally from about 0.5 to about 10% by weight of the therapeutic protein with about 1% being preferred, and from about 0.5 to about 20% by weight of the proteolytic enzyme inhibiting protein with about 10% being preferred. Again, the limitation of the upper range is a function of the viscosity of the coating material. The limitation of the lower range is a practical limitation.

If the protein solution has a low viscosity, it may be desirable to add 1-10% of polyvinylpyrollidone to bind the therapeutic protein to the nonpareil.

This aqueous solution of protein is then coated onto nonpareils. Nonpareils are small round particles of pharmaceutically inert materials.

Generally a nonpareil formed from the combination of sucrose and starch is preferred. One such brand is Nupareils which is sold by Ingredient Technology Corporation. The preferred size is 30-35 mesh.

The nonpareils are coated with 5-30% protein on a solids basis in a fluid bed spray applicator. Glatt brand powder coater granulators such as the GPCG-1, GPCG-5, or GPCG-60 fluid bed coaters are suitable for use in this application. Coating conditions and times will vary depending on the apparatus and coating viscosity. But, generally all coating steps must be conducted at less than 50°C. preferably less than 35°C. to avoid denaturing the protein.

The protein coated particles are dried and subsequently coated with an acid stable polymer (enteric coating). Generally, the coating will be applied in the same manner as the protein with the same equipment.

The coating composition used in the present invention is preferably a water based emulsion polymer. There are many of these which are on the market which are specifically used as enteric coatings. These include methacrylic acid copolymers sold under the trademark Eudragit L 30D manufactured by Rhom Pharma, cellulose acetate phthlate polymers sold under the trademark CAP manufactured by Kodak and hydroxy propyl methyl cellulose acetate succinate (HPMCAS) produced by Shin Etsu Chemical Company. Generally the coating composition is applied as 10-20% aqueous solution.

These compositions can be combined with a plasticizer to improve the continuity of the coating. There are several well known plasticizers typically used. Triethylcitrate (TEC) sold by Morfley Inc. is preferred. This can form about 2-3% of coating composition.

Talc (3.0% of coating composition) can also be added to prevent sticking between the particles if desired. Also an antifoaming agent (0.0025% of coating composition) such as sorbitan sesquioleate (Nikko Chemicals Company Limited) or silicone can be added.

It is important that the coating process be a totally aqueous process wherein the proteins as well as the coating composition itself are suspended in water. By avoiding using nonaqueous solvents, the therapeutic protein is unaffected during the coating process. An exemplary coating composition can include:
10% Shin-Etsu Aqoat AS-MF (HPMCAS);
2.8% TEC;
3.0% talc;
0.0025% sorbiton sesquioleate; and
balance water.

The coated particles then can be held in gel capsules and orally administered. The dosage of the therapeutic protein will be the same or higher as would be administered intravascularly. This dosage will depend on the individual and the course of the therapy. For example, with ragweed allergen, the dosage would start at .1 microgram per day and be increased up to 120 micrograms per day over 20 to 30 days.

As indicated, the stabilizing agent can also be mixed in dry form with the therapeutic protein and fillers such as starch or succrose. This can be put into a gel capsule (preferably an acid stable gel capsule) for oral administration. This embodiment is also shown in the example below.

The invention will be further appreciated in light of these following examples.

### Example 1

### Preservation of the Antigen During Encapsulation Testing by ELISA

30% acrylic coated particles containing 10% ovalbumin (OVA) and 1% ragweed by weight or uncoated particles of the same antigen makeup were incubated in 0.1M HCl, pH 1.2 or 0.2M phosphate buffer, pH 6.8 (1 gm particles/10 ml buffer). After incubations of 15 min. and 4 hours, supernatants were removed and held at 4°C. overnight prior to testing these by the inhibition assay.

Supernatants were diluted sequentially (1:2) in the wells of a gelatin blocked 96 well microtitre plate. An equal volume of a polyclonal rabbit anti-ragweed antisera (1:1000 dilution) was added to all wells. Inhibitor antisera mixtures were incubated for 2 hours at 37°C. prior to their transfer to ragweed extract coated ELISA plates. ELISA plates were developed using an alkaline phosphatase coupled goat antirabbit antisera and substrate.

Controls included the following:
1. anti-ragweed with no inhibitor added (positive control);
2. no anti-ragweed antisera (blanks); and
3. a mixture of OVA + ragweed extract as an inhibitor.

A standard consisted of dilutions of a ragweed extract used as an inhibitor.

### ELISA Results

Positive control (no inhibitor) at A406 = 1.141
Blanks (no anti-ragweed antisera) at A406 = 0.200

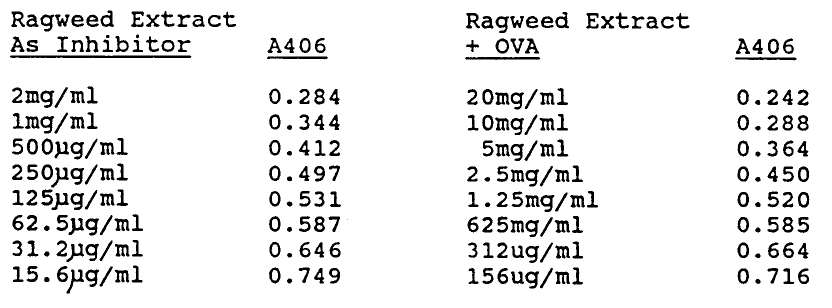

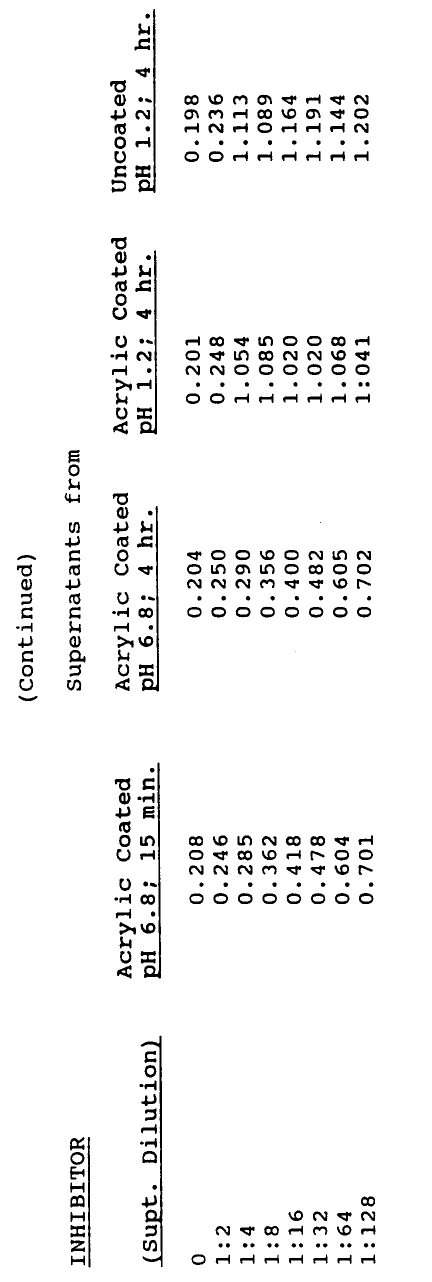

### Conclusions

1. Ragweed released from acrylic coated particles at pH 6.8 compete strongly for the binding to anti-ragweed antiserum with ragweed coated on ELISA plates.
2. The amount of ragweed released from acrylic coated particles at pH 6.8 after 15 minutes of incubation is similar to that released after 4 hours.
3. No antibody binding ragweed was released from particles (coated or uncoated) at pH 1.2. Lack of release indicated retention of ragweed proteins in the capsule at low pH and instability of the protein if left uncoated.
4. The presence of ovalbumin does not hinder the detection of ragweed by the inhibition assay.

### Example 2

### Antitrypsin Activity of OVA

Trypsin is one of the intestinal proteolytic enzymes responsible for the rapid degradation of ingested protein antigens. Ovalbumin is a naturally occurring inhibitor of trypsin. This characteristic of ovalbumin was examined in order to determine if its inclusion in acrylic coated particles might have a protective function for therapeutic proteins once they are released into the intestine.

Solutions containing various concentrations of ovalbumin or trypsin inhibitor (from soybean) were added to 2,000 IU of trypsin (2,000 U trypsin/1ml in 1mM HCl). After 15 minutes, 1ml aliquotes of these mixtures were transferred to tubes containing a solution of BAPNA (na-Benzoyl-DL-Arginine p-Nitoanalide). BAPNA is a chromogenic agent which absorbs light at 410nm following trypsin digestion.

### Results

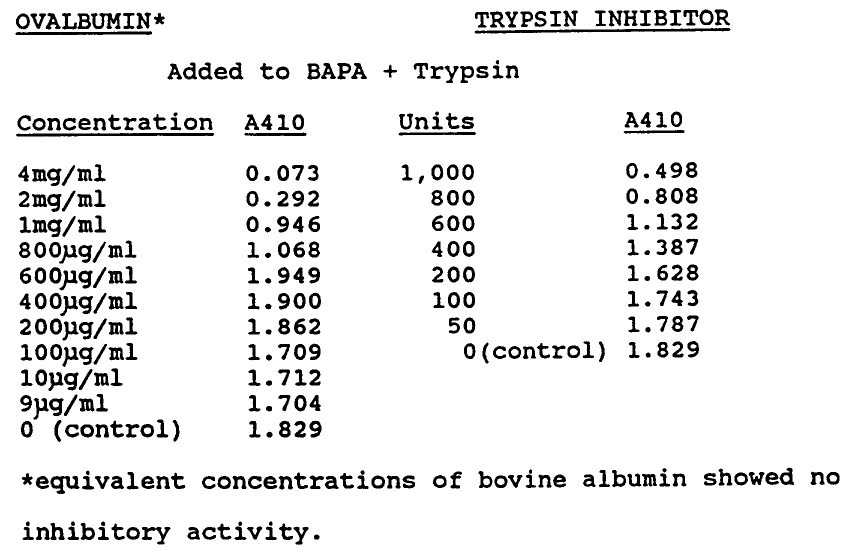

### Conclusion

Ovalbumin inhibited trypsin activity by preventing digestion of BAPNA at OVA concentration of 4, 2 and 1 mg/ml.

### Example 3

### Activity of Protein in Acrylic Coated Particles

The effectiveness of Eudragit L 30 D acrylic as an enteric coating for particles containing ragweed extract and ovalbumin was evaluated on the basis of a time-release assay for the protein performed at the gastric and intestinal pH and, for the ragweed antigens in particular by means of an ELISA inhibition assay. Coated particles suspended in 0.1M HCl pH 1.2 (simulated gastric solution) show a complete retention of the protein beyond two hours and less than a 20% protein release at four hours. Uncoated particles show no retention of the protein at the gastric pH. Suspension of particles in 0.2M phosphate buffer, pH 6.8 (simulated intestinal solution) resulted in a near complete release of the protein within 30 minutes.

### Example 4

### Immunogenicity of Encapsulated OVA and Ragweed

Immunological properties of OVA and ragweed released from microcapsules were tested following oral administration to BDF mice. Control groups of mice were immunized with the same antigens which were not encapsulated.

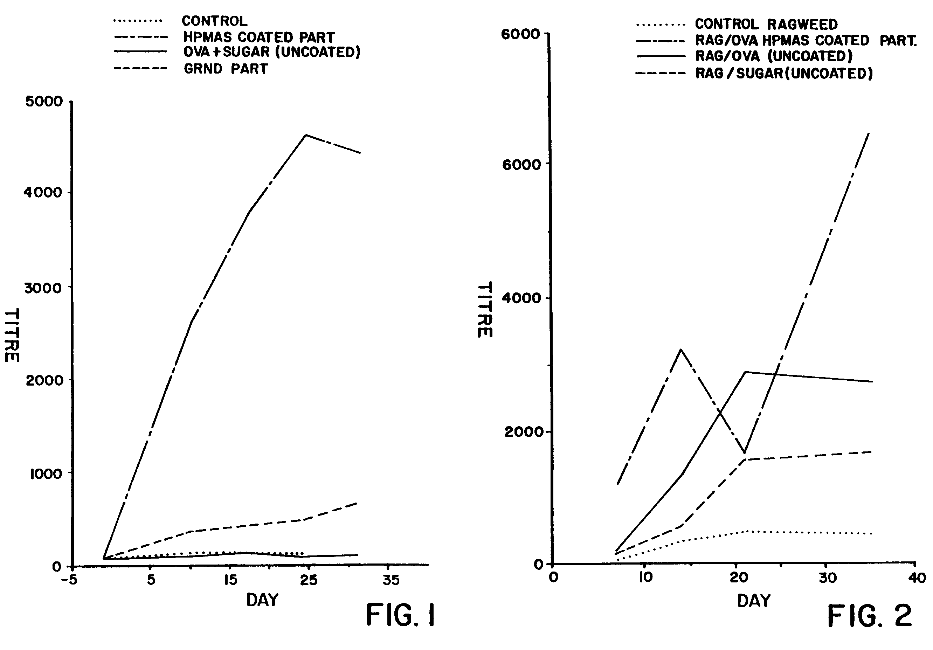

In the first set of experiments, encapsulated OVA was fed to BDF mice. Subsequently the mice were bled and their serum antibody levels determined by ELISA. As shown in Fig. 1, oral administration of encapsulated OVA resulted in significant immune response to the specific antigen. Unencapsulated antigen (OVA) was not immunogenic.

As shown in Fig. 2, when encapsulated OVA and unencapsulated OVA ragweed mixtures were orally administered to mice which were subsequently injected I.P. with the above antigens, immune response to encapsulated antigens was significantly higher than that of the unencapsulated proteins. Immune response to the encapsulated ragweed/OVA combination was significantly higher than to the unencapsulated combination or the ragweed.

These experiments demonstrate the importance of the many features of the present invention. The combination of the therapeutic protein with the stabilizing protein maintains the activity of the therapeutic protein. Coating this protein with an enteric coating protects it as it passes through the stomach. Further, the aqueous coating environment process protects the therapeutic protein during the process. Further, degradation of the therapeutic protein is prevented or reduced in the presence of a stabilizing protein. All of these act to provide an active orally administrable therapeutic protein composition.

## Claims

1. A method of forming a microencapsulated allergy treatment pharmaceutical, comprising forming an aqueous solution of a therapeutic protein, the therapeutic protein comprising an allergen involved in production of a tissue sensitizing immunoglobulin IgE antibody, coating the aqueous solution of protein onto inert particles and drying the particles, and applying an aqueous emulsion of an enteric coating onto the coated particles and subsequently drying the coated particles.

2. A method as claimed in Claim 1, wherein the allergen is selected from the group consisting of pollen allergens, dust, mites, bee venom, food allergens, animal dander, insect venom, and allergenic fragments.

3. A method as claimed in either Claim 1 or Claim 2, wherein the allergen is either pollen or allergenic fragments thereof.

4. A method as claimed in any preceding Claim, wherein the enteric coating is a methacrylic acid copolymer.

5. A pharmaceutical produced by a method as claimed in any preceding Claim.

6. A pharmaceutical as claimed in Claim 5, for use in treating allergy by oral administration of the pharmaceutical.

7. Use of a pharmaceutical as claimed in Claim 5, in the manufacture of medicament for treating allergy by oral administration of the pharmaceutical.

## Patentansprüche

1. Verfahren zur Herstellung eines mikroverkapselten Arzneimittels zur Behandlung von Allergie, bei dem man eine wäßrige Lösung eines therapeutischen Proteins herstellt, wobei das therapeutische Protein ein Allergen enthält, welches bei der Produktion eines gewebesensibilisierenden Immunglubolin-IgE-Antikörpers beteiligt ist, die wäßrige Lösung des Proteins auf inerte Teilchen aufbeschichtet und die Teilchen trocknet und auf die beschichteten Teilchen eine wäßrige Emulsion einer darmlöslichen Beschichtung aufbringt und die beschichteten Teilchen anschließend trocknet.

2. Verfahren nach Anspruch 1, bei dem das Allergen ausgewählt wird aus der Gruppe der Pollenallergene, Staub, Milben, Bienengift, Nahrungsmittelallergene, Tiergifte, Insektengifte und allergenischen Fragmenten.

3. Verfahren nach Anspruch 1 oder 2, bei dem das Allergen entweder Pollen oder allergenische Fragmente davon ist.

4. Verfahren nach einem der vorhergehenden Ansprüche, bei dem die darmlösliche Beschichtung ein Methacrylsäure-Copolymer ist.

5. Arzneimittel, hergestellt mit einem Verfahren gemäß einem der vorhergehenden Ansprüche.

6. Arzneimittel nach Anspruch 5 zur Anwendung bei der Behandlung von Allergie durch orale Verabreichung des Arzneimittels.

7. Verwendung eines Arzneimittels gemäß Anspruch 5 bei der Herstellung eines Medikaments zur Behandlung von Allergie durch orale Verabreichung des Arzneimittels.

## Revendications

1. Procédé de fabrication d'un produit pharmaceutique microencapsulé pour le traitement d'allergies comprenant :
- la préparation d'une solution aqueuse d'une protéine thérapeutique, cette protéine comprenant un allergène impliqué dans la production d'anticorps IgE sensibilisant les tissus,
- le revêtement de particules inertes par cette solution aqueuse de protéine et le séchage de ces particules, et
- l'application sur ces particules d'une émulsion aqueuse d'un revêtement gastro-résistant puis le séchage de ces particules recouvertes.

2. Procédé selon la revendication 1, dans lequel l'allergène est sélectionné dans le groupe constitué des allergènes de pollen, des poussières, des acariens, du venin d'abeille, des allergènes alimentaires, des phanères animaux, du venin d'insecte, des fragments allergènes.

3. Procédé selon l'un ou l'autre des revendications 1 et 2, dans lequel l'allergène est soit le pollen soit des fragments allergènes de pollen.

4. Procédé selon l'une quelconque des revendications précédentes, dans lequel le revêtement gastro-résistant est un copolymère d'acide méthacrylique.

5. Produit pharmaceutique fabriqué par un procédé selon l'une quelconque des revendications précédentes.

6. Produit pharmaceutique selon la revendication 5 destiné au traitement d'allergies par administration orale.

7. Utilisation d'un produit pharmaceutique selon la revendication 5 dans la fabrication de médicament pour le traitement d'allergies par administration orale de ce produit.
